(19) Europäisches Patentamt
European Patent Office
Office européen des brevets

(11) Veröffentlichungsnummer : **0 084 667**
**B1**

(12) **EUROPÄISCHE PATENTSCHRIFT**

(45) Veröffentlichungstag der Patentschrift : **18.09.85**

(21) Anmeldenummer : **82111928.6**

(22) Anmeldetag : **23.12.82**

(51) Int. Cl.⁴ : **C 07 C 63/66**, C 07 C 69/76, C 07 C103/78, C 07 D257/06, A 61 K 31/19, A 61 K 31/235, A 61 K 31/165, A 61 K 31/41

# (54) Phenylethylen-Derivate, ihre Herstellung und Verwendung als Arzneimittel.

(30) Priorität : **23.01.82 DE 3202100**
**23.01.82 DE 3202065**
**23.01.82 DE 3202118**

(43) Veröffentlichungstag der Anmeldung : **03.08.83 Patentblatt 83/31**

(45) Bekanntmachung des Hinweises auf die Patenterteilung : **18.09.85 Patentblatt 85/38**

(84) Benannte Vertragsstaaten :
**AT BE CH DE FR GB IT LI NL SE**

(56) Entgegenhaltungen :
**DE-A- 2 854 354**
**Chemical Abstracts Band 96, Nr. 17, 26. April 1982, Columbus, Ohio, USA W. BOLLAG "Arotinoids. A new class of retinoids with activities in oncology and dermatology", Seite 635, Spalte 1, Abstract Nr. 141529y**

(73) Patentinhaber : **BASF Aktiengesellschaft**
**Carl-Bosch-Strasse 38**
**D-6700 Ludwigshafen (DE)**

(72) Erfinder : **Frickel, Fritz-Frieder, Dr.**
**Silvanerweg 7**
**D-6705 Deidesheim (DE)**
Erfinder : **Nuerrenbach, Axel, Dr.**
**Koenigsberger Strasse 7**
**D-6718 Gruenstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 084 667 B1**

Jouve, 18, rue St-Denis, 75001 Paris, France

**Beschreibung**

Die Erfindung betrifft Phenylethylen-Derivate, Verfahren zu deren Herstellung sowie diese enthaltende therapeutische Mittel und diese Phenylethylen-Derivate zur Verwendung bei der Bekämpfung von Krankheiten.

Aus der DE-OS 2 854 354 ist bekannt, daß Stilben-Derivate pharmakologische Wirkungen bei der topischen und systemischen Therapie von Neoplasien, Akne, Psoriasis und anderen dermatologischen Affektionen aufweisen. Nachteilig ist jedoch vor allen Dingen die ausgeprägte toxische (Neben-) Wirkung dieser Verbindungen, die sie als Mittel in der topischen und systemischen Therapie von Neoplasien, Akne, Psoriasis und anderen dermatologischen Affektionen wenig geeignet erscheinen lassen. Die Nachteiligkeit der Stilben-Derivate der DE-OS 2 854 354 wird beispielsweise von A. Kistler in Calcified Tissue International 33, 249-254 (1981) beschrieben und offenbart sich insbesondere bei mehrmaliger Applikation an Nagern nach der von R. C. Moon et al [Cancer Research 39, 1339-1346 (1979)] publizierten Methode.

Der vorliegenden Erfindung liegt die Aufgabe zugrunde, Verbindungen mit vergleichbarer Wirkungsstärke, aber geringeren toxischen Nebenwirkungen zur Verfügung zu stellen.

Die Erfindung betrifft Phenylethylen-Derivate der Formel

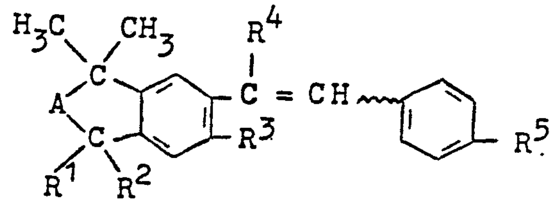

(I)

worin

A eine gegebenenfalls durch eine Methylgruppe substituierte Methylen- oder Ethylengruppe
$R^1$ ein Wasserstoffatom oder eine Methylgruppe,
$R^2$ ein Wasserstoffatom oder eine Methylgruppe,
$R^3$ ein Wasserstoffatom oder eine $C_{1-8}$-Alkylgruppe,
$R^4$ eine $C_{1-4}$-Alkylgruppe und
$R^5$ eine p-Hydroxyphenylenaminocarbonyl-, Tetrazol-5-yl-aminocarbonylgruppe oder — falls $R^3$ eine $C_{1-8}$-Alkylgruppe bedeutet — auch eine Carboxyl- oder $C_{2-4}$-Carbalkoxygruppe
darstellen, sowie gegebenenfalls deren Salze mit physiologisch verträglichen Basen.

Bevorzugt sind die Verbindungen, in denen A einen Ethylenrest, $R^1$, $R^2$ und $R^4$ Methylgruppen, $R^3$ eine $C_{1-8}$-Alkylgruppe und $R^5$ eine Carboxyl- oder $C_{2-4}$-Carbalkoxygruppe darstellen, sowie deren Salze mit physiologisch verträglichen Säuren.

Besonders wertvoll sind die Verbindungen, in denen die Phenylringe zueinander trans-ständig sind.

Die wellenförmig dargestellte C-C-Bindung kann vor oder hinter der Zeichenebene verlaufen und demzufolge zu einer cis-(Z-)-oder einer trans-(E)-Verbindung gehören.

Typische Beispiele für erfindungsgemäße Verbindungen sind :

4-[2-Methyl-2-(1,1,4,4,6-pentamethyl-1,2,3,4-tetrahydronaphth-7-yl)-vinyl]-benzoesäureethylester
4-[2-Methyl-2-(1,1,4,4,6-pentamethyl-1,2,3,4-tetrahydronaphth-7-yl)-vinyl]-benzoesäure
4-[2-Methyl-2-(1,1,6-trimethyl-1,2,3,4-tetrahydronaphth-7-yl)-vinyl]-benzoesäure
4-[2-Methyl-2-(1,1,6-trimethyl-1,2,3,4-tetrahydronaphth-7-yl)-vinyl]-benzoesäureethylester
4-[2-Methyl-2-(1,1,4,4-tetramethyl-6-ethyl-1,2,3,4-tetrahydronaphth-7-yl)-vinyl]-benzoesäureethylester
4-[2-Methyl-2-(1,1,4,4-tetramethyl-6-ethyl-1,2,3,4-tetrahydronaphth-7-yl)-vinyl]-benzoesäure
4-[2-Methyl-2-(1,1,4,4-tetramethyl-6-isopropyl-1,2,3,4-tetrahydronaphth-7-yl)-vinyl]-benzoesäure
4-[2-Methyl-2-(1,1,4,4-tetramethyl-6-isopropyl-1,2,3,4-tetrahydronaphth-7-yl)-vinyl]-benzoesäureethylester
4-[2-Methyl-2-(1,1,4,4-tetramethyl-6-propyl-1,2,3,4-tetrahydronaphth-7-yl)-vinyl]-benzoesäure
4-[2-Methyl-2-(1,1,4,4-tetramethyl-6-propyl-1,2,3,4-tetrahydronaphth-7-yl)-vinyl]-benzoesäureethylester
4-[2-Methyl-2-(1,1,4,4-tetramethyl-6-butyl-1,2,3,4-tetrahydronaphth-7-yl)-vinyl]-benzoesäuremethylester
4-[2-Methyl-2-(1,1,4,4-tetramethyl-6-butyl-1,2,3,4-tetrahydronaphth-7-yl)-vinyl]-benzoesäureethylester
4-[2-Methyl-2-(1,1,4,4-tetramethyl-6-butyl-1,2,3,4-tetrahydronaphth-7-yl)-vinyl]-benzoesäure
4-[2-Methyl-2-(1,1,4,4-tetramethyl-6-(2-methyl-propyl)-1,2,3,4-tetrahydronaphth-7-yl)-vinyl]-benzoesäureethylester
4-[2-Methyl-2-(1,1,4,4-tetramethyl-6-(2-methyl-propyl)-1,2,3,4-tetrahydronaphth-7-yl)-vinyl]-benzoesäure
4-[2-Methyl-2-(1,1,3,4,4,6-hexamethyl-1,2,3,4-tetrahydronaphth-7-yl)-vinyl]-benzoesäureethylester

4-[2-Methyl-2-(1,1,3,4,4,6-hexamethyl-1,2,3,4-tetrahydronaphth-7-yl)-vinyl]-benzoesäure
4-[2-Methyl-2-(1,1,2,3,3,5-hexamethyl-indan-6-yl)-vinyl]-benzoesäureethylester
4-[2-Methyl-2-(1,1,2,3,3,5-hexamethyl-indan-6-yl)-vinyl]-benzoesäurepropylester
4-[2-Methyl-2-(1,1,2,3,3,5-hexamethyl-indan-6-yl)-vinyl]-benzoesäureisopropylester
4-[2-Methyl-2-(1,1,2,3,3,5-hexamethyl-indan-6-yl)-vinyl]-benzoesäure
4-[2-Methyl-2-(1,1,3,3,5-pentamethyl-indan-6-yl)-vinyl]-benzoesäure
4-[2-Methyl-2-(1,1,3,3,5-pentamethyl-indan-6-yl)-vinyl]-benzoesäuremethylester
4-[2-Methyl-2-(1,1,3,3,5-pentamethyl-indan-6-yl)-vinyl]-benzoesäureethylester
4-[2-Methyl-2-(1,1,4,4,6-pentamethyl-1,2,3,4-tetrahydronaphth-7-yl)-vinyl]-benzoesäuremethylester
4-[2-Methyl-2-(1,1,4,4,6-pentamethyl-1,2,3,4-tetrahydronaphth-7-yl)-vinyl]-benzoesäurepropylester
4-[2-Methyl-2-(1,1,4,4-tetramethyl-6-n-butyl-1,2,3,4-tetrahydronaphth-7-yl)-vinyl]-benzoesäure
4-[2-Methyl-2-(1,1,4,4-tetramethyl-6-n-octyl-1,2,3,4-tetrahydronaphth-7-yl)-vinyl]-benzoesäure
4-[2-Methyl-2-(1,1,6-trimethyl-1,2,3,4-tetrahydronaphth-7-yl)-vinyl]-benzoesäure
4-[2-Methyl-2-(1,1,4,4-tetramethyl-1,2,3,4-tetrahydronaphth-7-yl)-vinyl]-N-(tetrazol-5-yl)-benzoesäureamid
4-[2-Methyl-2-(1,1,4,4,6-pentamethyl-1,2,3,4-tetrahydronaphth-7-yl)-vinyl]-N-tetrazol-5-yl)-benzoesäureamid
4-[2-Methyl-2-(1,1,4,4-tetramethyl-6-ethyl-1,2,3,4-tetrahydronaphth-7-yl)-vinyl]-N-(tetrazol-5-yl)-benzoesäureamid
4-[2-Methyl-2-(1,1,4,4-tetramethyl-6-isopropyl-1,2,3,4-tetrahydronaphth-7-yl)-vinyl]-N-(tetrazol-5-yl)-benzoesäureamid
4-[2-Methyl-2-(1,1,2,3,3-pentamethyl-indan-6-yl)-vinyl]-N-(tetrazol-5-yl)-benzoesäureamid
4-[2-Methyl-2-(1,1,3,3-tetramethyl-indan-6-yl)-vinyl]-N-(tetrazol-5-yl)-benzoesäureamid
4-[2-Methyl-2-(1,1,4,4-tetramethyl-6-propyl-1,2,3,4-tetrahydronaphth-7-yl)-vinyl]-N-(tetrazol-5-yl)-benzoesäureamid
4-[2-Methyl-2-(1,1,4,4-tetramethyl-6-(2-methyl-propyl)-1,2,3,4-tetrahydronaphth-7-yl)-vinyl]-N-(tetrazol-5-yl)-benzoesäureamid
4-[2-Methyl-2-(1,1,4,4-tetramethyl-6-butyl-1,2,3,4-tetrahydronaphth-7-yl)-vinyl]-N-(tetrazol-5-yl)-benzoesäureamid
4-[2-Methyl-2-(1,1-dimethyl-1,2,3,4-tetrahydronaphth-7-yl)-vinyl]-N-(tetrazol-5-yl)-benzoesäureamid
4-[2-Methyl-1,1,6-trimethyl-1,2,3,4-tetrahydronaphth-7-yl)-vinyl]-N-(tetrazol-5-yl)-benzoesäureamid
4-[2-Methyl-2-(1,1,3,3,5-pentamethyl-indan-6-yl)-vinyl]-N-(tetrazol-5-yl)-benzoesäureamid
4-[2-Methyl-2-(1,1,2,3,3,5-hexamethyl-indan-6-yl)-vinyl]-N-(tetrazol-5-yl)-benzoesäureamid
4-[2-Methyl-2-(1,1,4,4-tetramethyl-1,2,3,4-tetrahydronaphth-7-yl)-vinyl]-benzoesäure-(4-hydroxyanilid)
4-[2-Methyl-2-(1,1,4,4,6-pentamethyl-1,2,3,4-tetrahydronaphth-7-yl)-vinyl]-benzoesäure-(4-hydroxyanilid)
4-[2-Methyl-2-(1,1,4,4-tetramethyl-6-ethyl-1,2,3,4-tetrahydronaphth-7-yl)-vinyl]-benzoesäure-(4-hydroxyanilid)
4-[2-Methyl-2-(1,1,4,4-tetramethyl-6-isopropyl-1,2,3,4-tetrahydronaphth-7-yl)-vinyl]-benzoesäure-(4-hydroxyanilid)
4-[2-Methyl-2-(1,1,2,3,3-pentamethyl-indan-6-yl)-vinyl]-benzoesäure-(4-hydroxyanilid)
4-[2-Methyl-2-(1,1,3,3-tetramethyl-indan-6-yl)-vinyl]-benzoesäure-(4-hydroxyanilid)
4-[2-Methyl-2-(1,1,4,4-tetramethyl-6-propyl-1,2,3,4-tetrahydronaphth-7-yl)-vinyl]-benzoesäure-(4-hydroxyanilid)
4-[2-Methyl-2-(1,1,4,4-tetramethyl-6-(2-methyl-propyl)-1,2,3,4-tetrahydronaphth-7-yl)-vinyl]-benzoesäure-(4-hydroxyanilid)
4-[2-Methyl-2-(1,1,4,4-tetramethyl-6-butyl-1,2,3,4-tetrahydronaphth-7-yl)-vinyl]-benzoesäure-(4-hydroxyanilid)
4-[2-Methyl-2-(1,1-dimethyl-1,2,3,4-tetrahydronaphth-7-yl)-vinyl]-benzoesäure-(4-hydroxyanilid)
4-[2-Methyl-2-(1,1,6-trimethyl-1,2,3,4-tetrahydronaphth-7-yl)-vinyl]-benzoesäure-(4-hydroxyanilid)
4-[2-Methyl-2-(1,1,3,3,5-pentamethyl-indan-6-yl)-vinyl]-benzoesäure-(4-hydroxyanilid)
4-[2-Methyl-2-(1,1,2,3,3,5-hexamethyl-indan-6-yl)-vinyl]-benzoesäure-(4-hydroxyanilid).

Die erfindungsgemäßen Verbindungen weisen, soweit sie Derivate der freien Benzoesäure darstellen, ein acides Wasserstoffatom auf und können daher mit Basen in üblicher Weise in ein physiologisch verträgliches, gut wasserlösliches Salz übergeführt werden. Geeignete Salze sind beispielsweise Ammoniumsalze, Alkalimetallsalze — insbesondere des Natriums, Kaliums und Lithiums — Erdalkalimetallsalze — insbesondere des Calciums oder Magnesiums — sowie Salze mit geeigneten organischen Basen, wie mit niederen Alkylaminen — z. B. Methylamin oder Ethylamin — mit substitutierten niederen Alkylaminen, insbesondere hydroxysubstituierten Alkylaminen — wie Diethanolamin, Triethanolamin oder Tris-(hydroxymethyl)-aminomethan -, Piperidin oder Morpholin.

Besitzen die neuen Verbindungen einen Tetrazolrest, so können deren Alkali- und Erdalkalisalze hergestellt werden.

Die neuen Verbindungen werden hergestellt, indem man

a) eine Verbindung der Formel II

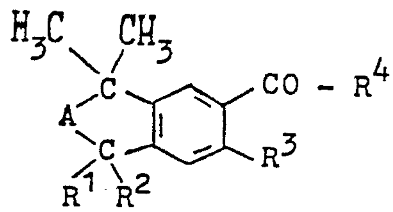

(II)

worin A und $R^1$-$R^4$ die in Anspruch 1 angegebene Bedeutung besitzen, mit einer Phosphorverbindung der Formel III

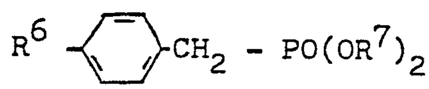

(III)

worin $R^6$ die für $R^5$ angegebene Bedeutung hat oder eine Cyanogruppe bedeutet und $R^7$ eine $C_{1-4}$-Alkylgruppe darstellt, nach Wittig-Horner umsetzt oder

b) ein Phosphoniumsalz der Formel IV

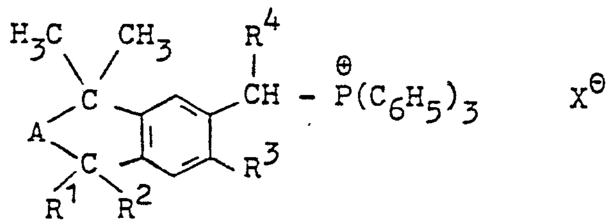

(IV)

worin A, $R^1$, $R^2$, $R^3$ und $R^4$ die in Anspruch 1 angegebene Bedeutung besitzen und X Chlor oder Brom bedeutet, mit einem Benzaldehyd-Derivat der Formel

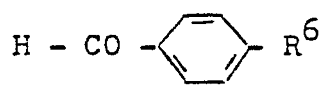

(V)

worin $R^6$ die oben angegebene Bedeutung besitzt, nach Wittig umsetzt

sowie anschließend — falls $R^6$ keine Carboxylgruppe darstellt — die so erhaltene Verbindung gegebenenfalls verseift und die so oder direkt erhaltenen freien Säuren anschließend gewünschtenfalls mit einem $C_{1-3}$-Alkohol, p-Hydroxyanilin oder 5-Aminotetrazol umsetzt und die so erhaltenen Verbindungen — falls gewünscht — in ihre Salze mit physiologisch verträglichen Basen überführt.

Die Umsetzungen a) und b) verlaufen bei einer Temperatur von bis zu 100 °C, zweckmäßig bei 20 bis 50 °C. Die Umsetzungen können bei atmosphärischem Druck oder in einem geschlossenen Gefäß unter erhöhtem Druck, gegebenenfalls unter Erwärmung auf den angegebenen Temperaturbereich durchgeführt werden.

Man kann diese Umsetzungen in Gegenwart eines Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man cyclische Ether, wie Dioxan oder Tetrahydrofuran sowie insbesondere Dimethylformamid oder deren Mischungen, wobei die Umsetzung im allgemeinen bei einer Temperatur bis zu 30 °C abläuft.

Die Umsetzungen werden in Gegenwart eines Deprotonierungsmittels für die Phosphorverbindung (III) vorgenommen. Geeignet sind Alkalimetallhydride und Alkalimetallamide, insbesondere des Natriums und Kaliums, die Natrium- und Kaliumsalze von Dimethylsulfoxid, Alkyllithiumverbindungen wie n-Butyllithium oder Alkalimetallalkoholate, vorzugsweise Natriummethanolat und Natriumethanolat.

Bei Verwendung einer aliphatischen Epoxiverbindung, vorzugsweise Butylenoxid, gelingt die Reaktion ohne Zusatz weiterer Mittel. Aliphatische Epoxiverbindungen sind somit Lösungsmittel und Deprotonierungsmittel zugleich. Bei Verwendung von Butylenoxid als aliphatische Epoxiverbindung kann man die Umsetzung bei der Siedetemperatur des Reaktionsgemisches oder bei einer Temperatur um 100 °C in einem geschlossenen Gefäß unter erhöhtem Druck durchführen.

Die Umsetzung der Säure I ($R^5$ = COOH) mit einem $C_{1-3}$-Alkohol, p-Hydroxyanilin oder 5-Aminotetrazol gelingt durch Überführen der Säure in ein aktiviertes Derivat der Formel I, worin R den Rest COX mit X in der Bedeutung einer Abgangsgruppe darstellt.

X stellt einen Säurerest, z. B. ein Halogenatom — insbesondere Chlor oder auch Brom — oder den N-Oxy-succinimid-Rest dar.

Die Umsetzung verläuft bei einer Temperatur von bis zu 50 °C bei Atmosphärendruck oder in einem geschlossenen Gefäß unter erhöhtem Druck.

Man kann diese Umsetzung in Gegenwart eines Vedünnungsoder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man lineare oder cyclische Ether, wie Diethylether oder Tetrahydrofuran sowie insbesondere Dimethylformamid, wobei die Umsetzung im allgemeinen bei einer Temperatur bis zu 30 °C abläuft.

Üblicherweise wird die Umsetzung in Gegenwart einer Base als säurebindendem Mittel vorgenommen. Geeignete Basen sind Alkalimetallcarbonate, -hydrogencarbonate, insbesondere des Natriums and Kaliums, organische tertiäre Basen, wie Pyridin oder niedere Trialkylamin, wie Trimethyl- oder Triethylamin. Dabei wird die verwendete Base im Verhältnis zum eingesetzten Benzoesäurehalogenid in stöchiometrischer Menge oder in geringem Überschuß verwendet.

Eine andere Möglichkeit zur Herstellung der erfindungsgemäßen Säurederivate geht von den entsprechenden freien Säuren I ($R^5$ = COOH) aus ; man setzt diese in Gegenwart eines die Carboxylgruppe aktivierenden, wasserabspaltenden Mittels in einem Lösungsmittel mit einem $C_{1-3}$Alkohol, p-Aminophenol oder Tetrazolamin um.

Als wasserabspaltende aktivierende Reagentien können die bei der Peptidsynthese üblichen Reagentien verwendet werden (vgl. « The Peptides », Band I, Academic Press, N.Y., 1965, Seiten 77 bis 128). Das allgemeine Prinzip der Umsetzung besteht in der Aktivierung der Carboxylgruppe, beispielsweise durch Behandlung mit einem Carbodiimid, wie N,N'-Dicyclohexylcarbodiimid, oder durch intermidiäre Bildung das Säureazids, eines gemischten Anhydrids (beispeilsweise mit Kohlensäuremonoestern), eines aktivierrten Esters (beispielsweise des p-Nitrophenylesters) oder eines heterocyclischen Amids (beispielsweise eines Imidazolids) der entsprechenden Benzoesäure.

Die Behandlung einer an der Carboxylgruppe aktivierten Verbindung mit einem $C_{1-3}$-Alkohol, p-Aminophenol oder Tetrazolamin führt dann zum erfindungsgemäßen Säurederivat. Die Aktivierungs- und Verknüpfungsreaktionen können in Lösungsmitteln, vorzugsweise in N,N-Dimethylformamid, Tetrahydrofuran, Dioxan, Methylenchlorid, Nitromethan, Acetonitril, Dimethylsulfoxid, N,N-Dimethylacetamid und Hexamethylphosphorsäuretriamid vorgenommen werden. Eine geeignete Temperatur für beide Stufen, d. h. der Reaktion der Säure mit dem Kupplungsmittel und die Reaktion des aktivierten Zwischenproduktes mit p-Amino-phenol liegt zwischen 20 und 100 °C. Man kann dabei entweder stufenweise vorgehen, indem man das aktivierte Zwischenprodukt vor der Zugabe des p-Aminophenols isoliert oder vorteilhaft, indem man die Reaktionspartner nacheinander ohne Isolierung von Zwischenstufen zur Reaktion bringt.

Bei einer bevorzugten Verknüpfungsmethode verwendet man N,N-Carbonyldiimidazol und arbeitet in Dimethylformamid, wobei die Reaktionstemperatur für beide Stufen bei 20 bis 60 °C liegt.

Die Verbindungen der Formel I können einheitlich die cis-oder trans-Struktur haben oder Mischungen der cis-trans-Isomeren darstellen. Ein Gemisch kann durch HPLC-Analyse oder durch ein $^{13}$C-NMR-Spektrum quantitativ bestimmt und das jeweils gewünschte Isomere gegebenenfalls durch fraktionierende Krisallisation oder Chromatographie mit beispielsweise Kieselgelsäulen oder durch präparative HPL-Chromatographie isomerenrein isoliert werden.

Die erfindungsgemäßen Verbindungen und ihre physiologisch verträglichen Salze können zur topischen und systemischen Therapie und Prophylaxe von benignen und malignen Neoplasien und prämalignen Läsionen — z. B. Praekanzerosen und Karzinomen der Haut, der Schleimhäute und innerer Organe — sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen die prophylaktische und therapeutische Behandlung von Praekanzerosen und Tumoren. Von Vorteil ist dabei ihre geringe Toxizität.

Die pharmakologischen Wirkungen können beispielsweise in den nachfolgenden Testmodellen aufgezeigt werden :

1. Aufhebung der Keratinisierung in Trachealkulturen zum Nachweis der Antitumorwirkung :

Im Testmodell wird die intrinsische Eigenschaft der erfindungsgemäßen Verbindungen, die Differenzierung von Epithelialzellen zu steigern, bestimmt. Die hohe Signifikanz dieser Screeningsmethode bei der Vorhersage der potentiellen Verwendung eines neuen Retinoids zur Prevention von Tumoren im Epithelgewebe wird allgemein anerkannt. Dabei ist bekannt, daß jedes in vitro Testsystem mit Nachteilen behaftet ist, wenn es um die Vorhersage der in vivo Aktivität geht. Abgesehen von diesen grundsätzlichen

Einschränkungen ist das System der Trachealzellkulturen eine der wertvollsten Methoden, um die biologische Aktivität neuer Retinoide zu ermitteln.

Es wird die Fähigkeit der Testsubstanzen bestimmt, die Keratinisierung in einem definierten in vitro System aufzuheben. Tracheen von Hamstern, die sich in einem sehr frühen Zustand eines Vitamin A-Mangels befinden, wurden in Kultur genommen. Zum Zeitpunkt der Trachea-Entnahme waren die Tiere 29 bis 30 Tage alt (nachdem man sie nach 21 Tagen entwöhnt hatte) und sie ziegten immer noch eine Gewichtszunahme. Ihr Durchschnittsgewicht betrug 47 bis 52 g. Das tracheale Epithel war im allgemeinen ein schwaches Säulen- oder Pflaster-epithel mit nur vereinzelten Stellen squamöser Metaplasien. Jede Trachea wurde vom Kehlkopf bis zur Carina entlang der membranartigen Dorsalwand geöffnet und in serum-freiem Medium (CMRL-1066 ergänzt durch kristallines Rinderinsulin, 1,0 µg/ml, Hydrocortisonhemisuccinat, 0,1 µg/ml ; Glutamin 2 mM ; Penicillin, 100 Einheiten/ml, und Streptomycin, 100 µg/ml) in Kultur genommen. Die Kulturen wurden mit einem Gemisch aus 50 % Sauerstoff, 45 % Stickstoff und 5 % Kohlendioxid begast. Die Kulturschalen wurden leicht geschwenkt, um die Tracheen mit Gas und Kulturenmedium in Kontakt zu bringen. Alle Tracheen wurden zunächst 3 Tage ohne Retinoid-Zusatz in dem Kulturmedium gehalten. Nach 3 Tagen wurden einige Tracheen entnommen. Sie zeigten fast alle deutliche squamöse Metaplasien. Die verbleibenden Tracheen wurden in Gruppen eingeteilt, die dann mit folgenden Zusätzen behandelt wurden :

a) Testsubstanz in spektroskopisch reinem Dimethylsulfoxid gelöst ; die endgültige Konzentration an Dimethylsulfoxid im Kulturmedium war niemals größer als 0,1 %.

b) Die äquivalente Menge Dimethylsulfoxid ohne weiteren Zusatz.

Das Nährmedium wurde dreimal wöchentlich gewechselt. Alle verbliebenen Tracheen wurden nach 10 Tagen in Kultur aufgearbeitet. Die Tracheen wurden in 10 %iger gepufferter Formaldehydlösung fixiert und in Paraffin eingebettet. Schnitte von 5 µm durch das Zentrum wurden mit Hexatoxylin und Eosin angefärbt und unter dem Mikroskop auf die Anwesenheit von Keratin und Keratohyalin untersucht ; beides wurde in etwa 90 % aller ohneTestsubstanz gehaltenen Kontrollkulturen beobachtet. Dosis-Wikrungskurven der erfindungsgemäßen Verbindungen wurden aufgenommen. In der nachfolgenden Tabelle 1 werden die extrapolierten molaren Dosen angegeben, die in der Hälfte der Kulturen die Keratinisierung unterbinden (ED 50 %).

Tabelle 1

| Substanz des Beispiels | $ED_{50}$ [Mol/1] |
|---|---|
| 2 | $1.10^{-11}$ |
| 7 | $1.10^{-11}$ |
| 18 | $3.10^{-12}$ |
| 19 | $1.10^{-12}$ |
| 26 | $1.10^{-12}$ |
| Vitamin A-Säure | $1.10^{-11}$ |

Darüber hinaus induzieren die erfindungsgemäßen Verbindungen (insbesondere die Verbindung des Beispiels 2) die Zelldifferenzierung zu ausgereiften Granulozyten in Leukämiezellen von Patienten mit promeolozytischer Leukämie.

2. Die antiarthritische Wirkung der erfindungsgemäßen Verbindungen kann in üblicher Weise im Tierexperiment im Adjuvans-Arthritis-Modell bestimmt werden.

3. Die dermatologische Aktivität, beispielsweise für die Behandlung von Akne, kann u.a. durch die komedolytische Aktivität nachgewiesen werden.

Die Ausbildungen von Komedos wurde durch einmalige topische Applikation (0,5 ml pro Tag) an 5 % Teer in Polyan(R) (Ester von Lanolinalkohol und Linolensäure, Herst. Amerchol Corp., USA) auf beide Ohren von Albinokaninchen an 5 aufeinanderfolgenden Tagen pro Woche über zwei Wochen induziert. Anschließend erfolgt die topische Behandlung mit den Testsubstanzen in Ethanol : Propylenglykol (70 : 30 ; v/v ; 0,5 ml), die auf die innere Hautoberfläche eines Ohres jedes Kaninchens einmal täglich an 5 aufeinanderfolgenden Tagen pro Woche über zwei Wochen lang aufgetragen wurden. Das zweite Ohr jedes Tieres diente als unbehandelte Kontrolle.

Nach der sich noch anschließenden Behandlung (72 Stunden) mit der Prüfsubstanz wurden die Kaninchen getötet. Eine Hautprobe von ungefähr 6 $cm^2$ aus jeder Ohrmuschel gerade außerhalb des Gehörganges wurde entfernt und diese in ca. 1 $cm^2$ große Scheiben zerteilt. Diese Hautpartien wurden 2 Minuten in 60 °C warmes Wasser getaucht. Nach dem vorsichtigen Abschälen der Epidermis mit dem flachen Ende eines Spatels und einer kleinen Pinzette brachte man diese mit der dermalen Seite nach oben auf den Objektträger. Nach der Lufttrocknung über Nacht wurden die Prüfstreifen unter dem Stereomikroskop beurteilt. Follikulare Partien mit hornigem Material bleiben intakt. Die Komedone sind erkennbar als diskrete, gleichgestaltige, zylindrische bis runde Hornteile, deren Größe und Zahl proportional zur Aktivität der geprüften Substanz ist. Der komedolytische Effekt wurde bestimmt als die Abnahme der Komedozahl in % im Vergleich zum Kontrollohr, vgl. Tabelle 2.

Tabelle 2

| Substanz des Beispiels | komedolytische Aktivität in % |
|---|---|
| 1 | 75 |
| 2 | 78 |
| 5 | 54 |
| 6 | 68 |
| 7 | 59 |
| 8 | 62 |
| Vitamin A-Säure | 67 |

4. Als weiteres Maß für die dermatologische Aktivität diente der Nachweis der Fähigkeit die Anzahl der Utriculi im Modell der Rhino-Maus zu reduzieren. Diese Methode ist von L. H. Kligman et al in The Journal of Investigative Dermatology, 73, 354-358 (1979) beschrieben. Dabei werden an Rhino-Mäusen vorhandene Zysten, die als genetisch bedingte Läsionen der Haut vorhanden sind, sog. Akne-Zysten, durch Wirksubstanzgabe zurückgebildet und die Abnahme in % ausgedrückt.

Tabelle 3

| Substanz des Beispiels | Abnahme in % |
|---|---|
| 1 | 65 |
| 2 | 68 |
| 5 | 69 |
| 6 | 59 |
| 7 | 62 |
| 8 | 52 |
| Vitamin A-Säure | 60 |

5. Die Verträglichkeit der Testsubstanzen nach topischer Applikation wurde in Versuchen mit jeweils 6 weißen männlichen Neuseeland-Kaninchen bestimmt. Bei jedem Testtier wurden jeweils ungefähr 6 $cm^2$ große Rückenpartien rasiert. Nach dem Lösen der Prüfsubstanzen in Ethanol : Propylenglykol (70 : 30, v/v) wurden 0,2 ml davon mit einer automatischen Mikropipette an 9 aufeinanderfolgenden Tagen durch vorsichtiges Einreiben an einer bestimmten Stelle zweimal täglich in 6-stündigen Intervallen appliziert.

Alle Testflächen wurden vor jeder Applikation am Morgen subjektiv auf Erytheme und Abschuppung beurteilt. Man benutzte eine numerisch abgestufte Skala von 0 bis 3 (0 = keine Reaktion, 1 = mild, 2 = mittel, 3 = schwer). Der Mittelwert an Erythemen-Bildung und Abschuppung gibt die relative Irrationsfähigkeit der Prüfsubstanzen im Verlgeich zu Vitamin A-Säure wieder.

Tabelle 4

| Substanz des Beispiels | Erythem | Abschuppung |
|---|---|---|
| 2 | 2,8 | 2,7 |
| 5 | 2,0 | 1,7 |
| 7 | 3,0 | 3,0 |
| 8 | 1,4 | 1,0 |
| Vitamin A-Säure | 3,0 | 3,0 |

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise beispeilsweise durch Vermischen des Wirkstoffs mit den an sich in solchen Präparaten üblichen festen oder flüssigenTräger- und Hilfsstoffen.

Die Mittel können dementsprechend peroral, parenteral oder topsich verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei

systemischer Anwendung vorzugsweise in einer Einzeldosis von 0,1 bis 50 mg enthalten und täglich in einer oder mehreren Dosierungen je nach Art und Schwere der Erkrankung verabreicht werden.

Üblicherweise verwendete pharmazeutische technische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxyltoluol oder gesmacksverbessernde Zusätze, Stabilisierungsmittel, Emulgiermittel, Gleitmittel usw.zugesetzt werden. Voraussetzung ist, daß alle bei der Herstellung pharmazeutische Zubereitung verwendeten Stoffe toxikologisch unbedenklich sind und mit den verwendeten Wirkstoffen verträglich sind.

Die folgenden Beispiele erläutern die Herstellung der erfindungsgemäßen Verbindungen

## Beispiel 1

(E)-4-[2-Methyl-2-(1,1,4,4,6-pentamethyl-1,2,3,4-tetrahydronaphth-7-yl)-vinyl]-benzoesäureethylester

Zu einer Suspension von 250 ml Dimethylsulfoxid und 9 g 80 %iges Natriumhydrid, das zuvor mit Petrolether vom 20 %igen Paraffin-Anteil befreit worden war, wurde im Verlauf von 0,5 h eine Lösung von 90 g p-Carboxyethyl-benzyl-phosphonsäurediethylester in 150 ml DMSO bei 35 °C zugetropft. Anschließend wurde noch 2 h bei 40 °C gerührt und innerhalb von 10 min eine Lösung von 36,6 g 7-Acetyl-1,1,4,4,6-pentamethyltetralin in 70 ml Dimethylsulfoxid zugetropft.

Nach Stehen über Nacht wurde der Ansatz mit 100 ml Ethanol versetzt, auf 2 l Eis-Wasser gegossen und mit 2 N Salzsäure angesäuert. Der entstandene Niederschlag wurde abfiltriert und auf dem Filter mit 150 ml Ethanol gespült und mit 75 ml Methanol gewaschen. Es verblieben 35 g (E)-4-[2-Methyl-2-(1,2,4,4,6-pentamethyl-1,2,3,4-tetrahydronaphth-7-yl)-vinyl]-benzoesäureethylester, Schmp. 108 bis 109 °C.

Die HPLC-Analyse (Si 60 5 µm, 150 bar, n-Heptan/Essigsäureethylester (98 : 2), $t_R$ = 3,1 min) zeigte, daß die Verbindung zu über 98 % aus einem Isomeren bestand.

$^{13}$C-NMR-Spektrum ($CDCl_3$, Angaben in ppm) :

(Die Zählweise der C-Atome wurde zur Zuordnung der NMR-Signale willkürlich vorgenommen)

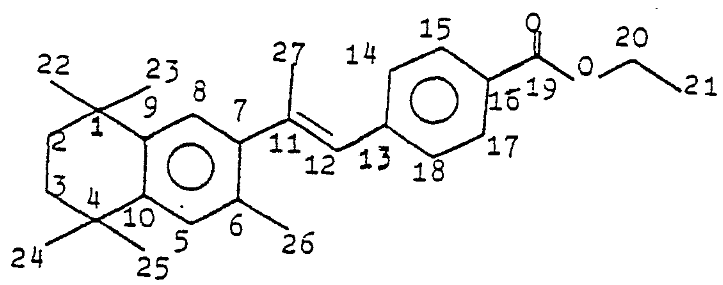

14,40 ($C_{21}$) ; 19,74 ($C_{27}$) ; 20,35 ($C_{26}$) ; 31,96 ($C_{25}$, $C_{24}$, $C_{23}$, $C_{22}$) ; 34,02 ($C_1$, $C_4$) ; 35,32 ($C_2$, $C_3$) ; 60,94 ($C_{20}$) ; 126,11 ($C_8$) ; 128,41 ($C_{16}$, $C_{12}$) ; 128,55 ($C_5$) ; 129,00 ($C_{14}$, $C_{18}$) ; 129,68 ($C_{15}$, $C_{17}$) ; 131,72 ($C_6$) ; 141,98 ($C_{13}$) ; 142,93 ($C_{11}$) ; 143,11 ($C_{10}$) ; 143,83 ($C_9$) ; 166,80 ($C_{19}$) ;

Das Signal bei 19,74 ($C_{27}$) belegt die E-(Trans-)-Geometrie der Verbindung.

## Beispiel 2

4,7 g (E)-4-[2-Methyl-2-(1,1,4,4,6-pentamethyl-1,2,3,4-tetrahydronaphth-7-yl)-vinyl]-benzoesäureethylester wurden mit 1,7 g 86 %igem Kaliumhydroxid in einem Gemisch aus 100 ml Ethanol und 5 ml Wasser 6 h bei 80 °C gerührt. Nach dem Überführen der gesamten Reaktionsmasse in 1 l Wasser wurde mit 2 N Salzsäure angesäuert, der erhaltene farblose Niederschlag abfiltriert und nach dem Trocknen auf der Nutsche mit 100 ml Heptan gewaschen. Nach dem Trocknen verblieben 4,0 g (E)-4-[2-Methyl-2-(1,1,4,4,6-pentamethyl-1,2,3,4-tetrahydronaphth-7-yl)-vinyl]-benzoesäure, Schmp. 206 °C.

$^{13}$C-NMR-Spektrum ($d_6$-DMSO, Angaben in ppm)

(Die Zählweise der C-Atome wurde zur Zuordnung der NMR-Signale willkürlich vorgenommen).

(Siehe Figur Seite 9 f.)

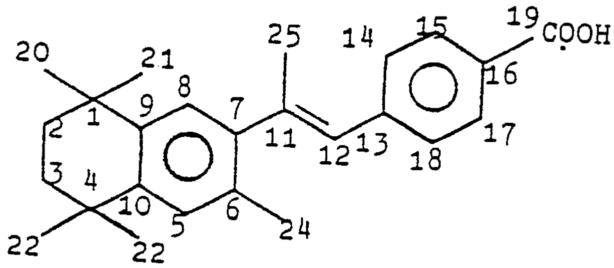

19,36 ($C_{25}$) ; 20,17 ($C_{24}$) ; 31,60 ($C_{20}$, $C_{21}$, $C_{22}$, $C_{23}$) ; 33,51 ($C_2$, $C_3$) ; 34,71 ($C_4$, $C_1$) ; 125,40 ($C_8$) ; 128,00 ($C_5$, $C_{12}$) ; 128,61 ($C_{16}$) ; 128,95 ; 129,42 ($C_{14}$, $C_{18}$) ; 129,42 ($C_{15}$, $C_{17}$) ; 131,12 ($C_6$) ; 141,20 ($C_{13}$) ; 141,70 ($C_7$) ; 142,16 ($C_{10}$) ; 142,52 ($C_{11}$) ; 143,08 ($C_9$) ; 167,26 ($C_{19}$).

Das Signal bei 19,36 ($C_{25}$) belegt die E-(trans-)-Geometrie der Verbindung.

## Beispiel 3

### A. Herstellung des Ausgangsmaterials

Aus 9 g 80 %igem Natriumhydrid, das zunächst mit Petrolether vom 20 %igen Paraffin-Anteil befreit worden war, wurde eine Suspension in 100 ml Dimethylsulfoxid hergestellt und innerhalb 0,5 h eine Lösung von 75,9 g p-Cyano-benzylphosphonsäurediethylester in 150 ml Dimethyl-sulfoxid bei ca. 35 °C zugetropft. Es wurde noch 2 h bei 40 °C gerührt und innerhalb von 10 min eine Lösung von 53,5 g 7-Acetyl-1,1,4,4,6-pentamethyltetralin in 50 ml Dimethylsulfoxid zugetropft.

Am folgenden Tag wurde das Reaktionsgemisch auf 3 l Eiswasser gegossen und mit 2 N Salzsäure angesäuert. Der entstandene Feststoff wurde abfiltriert und auf dem Filter mit 75 ml Methanol gewaschen. Nach dem Trocknen verblieben 30,2 g (E)-4-[2-Methyl-2-(1,1,4,4,6-pentaethyl-1,2,3,4-tetrahydronaphth-7-yl)-vinyl]-benzonitril, Schmp. 140 bis 141 °C.

Die HPLC-Analyse (Si 60 5 µm, 150 bar, 25 cm-Säule, n-Heptan/Essigsäureethylester (97 : 3), $t_R$ = 3,2 min) weist eine Isomerenreinheit von mehr als 95 % aus.

### B. Herstellung des Endprodukts

13,5 g (E)-4-[2-Methyl-2-(1,1,4,4,6-pentamethyl-1,2,3,4-tetrahydronaphth-7-yl)-vinyl]-benzonitril wurden in einem Gemisch aus 200 ml Ethanol und 200 ml 10 N Natronlauge 3 h auf Siedetemperatur erhitzt. Nach dem Abkühlen goß man auf 1 l Wasser, saugte den farblosen Niederschlag ab und erhielt nach dem Trocknen 14,9 g (E)-4-[2-Methyl-2-(1,1,4,4,6-pentamethyl-1,2,3,4-tetrahydronaphth-7-yl)-vinyl]-benzoesäure, Schmp. 204 bis 206 °C, vgl. Beispiel 2.

Die in der nachfolgenden Tabelle angegebenen Verbindungen wurden entweder durch Wittig-Horner-Reaktion oder durch Verseifung der entsprechenden Ester oder Nitrile hergestellt.

(Siehe Tabelle Seite 10 ff.)

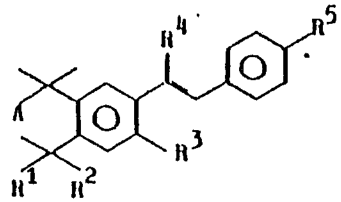

| Nr. | A | $R^1$ | $R^2$ | $R^3$ | $R^4$ | $R^5$ | Fp [°C] | Stereochemie | Wert der chem. Verschiebung ($^{13}C$-NMR) von $R^4$ [ppm] | Name |
|---|---|---|---|---|---|---|---|---|---|---|
| 4 | $-CH_2CH_2-$ | $-CH_3$ | $-CH_3$ | $-C_2H_5$ | $-CH_3$ | $-COOC_2H_5$ | 100-102 | E | 20,96 | (E)-4-[2-Methyl-2-(1,1,4,4--tetramethyl-6-ethyl-1,2,3,4--tetrahydronaphth-7-yl)-vinyl]-benzoesäureethylester |
| 5 | $-CH_2-CH_2-$ | $-CH_3$ | $-CH_3$ | $-C_2H_5$ | $-CH_3$ | $-COOH$ | 177-178 | E | 20,77 | (E)-4-[2-Methyl-2-(1,1,4,4--tetramethyl-6-ethyl--1,2,3,4-tetrahydronaphth--7-yl)-vinyl]-benzoesäure |
| 6 | $-CH_2-CH_2-$ | $-CH_3$ | $-CH_3$ | $-CH(CH_3)_2$ | $-CH_3$ | $-COOC_2H_5$ | 156-157 | E | 21,43 | (E)-4-[2-Methyl-2-(1,1,4,4-tetramethyl-6-isopropyl-1,2,3,4-tetrahydronaphth-7-yl)-vinyl]-benzoesäureethylester |
| 7 | $-CH_2-CH_2-$ | $-CH_3$ | $-CH_3$ | $-CH(CH_3)_2$ | $-CH_3$ | $-COOH$ | 237-238 | E | 21,22 | (E)-4-[2-Methyl-2-(1,1,4,4-tetramethyl-6-isopropyl-1,2,3,4-tetrahydronaphth-7-yl)-vinyl]-benzoesäure |
| 8 | $-CH_2-CH_2-$ | $-CH_3$ | $-CH_3$ | $-CH_2-CH(CH_3)_2$ | $-CH_3$ | $-COOH$ | 228-229 | E | 21,03 | (E)-4-[2-Methyl-2-(1,1,4,4-tetramethyl-6-(2-methylpropyl)-1,2,3,4-tetrahydronaphth-7-yl)-vinyl]-benzoesäure |
| 9 | $-CH_2-CH(CH_3)-$ | $-CH_3$ | $-CH_3$ | $-CH_3$ | $-CH_3$ | $-COOC_2H_5$ | 105 | E | 20,05 | (E)-4-[2-Methyl-2-(1,1,3,4,4,6-hexamethyl-1,2,3,4-tetrahydronaphth-7-yl)-vinyl]-benzoesäure-ethylester |

(Fortsetzung)

| Nr. | A | $R^1$ | $R^2$ | $R^3$ | $R^4$ | $R^5$ | Fp [°C] | Stereo-chemie | Wert der chem. Verschiebung ($^{13}C$-NMR) von $R^4$ [ppm] | Name |
|---|---|---|---|---|---|---|---|---|---|---|
| 10 | $-CH_2-CH_2-$ | $-CH_3$ | $-CH_3$ | $-CH_3$ | $-CH_3$ | -COOH | 276-277 | Z | 18,48 | (Z)-4-[2-Methyl-2-(1,1,4,4,6-pentamethyl-1,2,3,4-tetrahydronaphth-7-yl)-vinyl]-benzoesäure |
| 11 | $-CH_2-CH_2-$ | $-CH_3$ | $-CH_3$ | $-CH_2-CH_2-CH_3$ | $-CH_3$ | -COOH | 198-199 | E | 22,04 | (E)-4-[2-Methyl-2-(1,1,4,4-tetramethyl-6-propyl-1,2,3,4-tetrahydronaphth-7-yl)-vinyl]-benzoesäure |
| 12 | $-CH_2-CH(CH_3)-$ | $-CH_3$ | $-CH_3$ | $-CH_3$ | $-CH_3$ | -COOH | 254 | E | 20,11 | (E)-4-[2-Methyl-2-(1,1,3,4,4,6-hexamethyl-1,2,3,4-tetrahydronaphth-7-yl)-vinyl]-benzoesäure |
| 13 | $-CH_2-CH_2-$ | $-CH_3$ | $-CH_3$ | $-CH_3$ | $-CH_2-CH_3$ | -COOH | 252 | Z | 32,79 ($-CH_2-$) | ~~(E)~~ (Z)-4-[2-Ethyl-2-(1,1,4,4-tetramethyl-1,2,3,4-tetrahydronaphth-7-yl)-vinyl]-benzoesäure |
| 14 | $-CH_2-CH_2-$ | $CH_3$ | $CH_3$ | n-Bn | $CH_3$ | COOH | 237-240 | E | | (E)-4-[2-Methyl-2-(1,1,4,4-tetramethyl-6-n-butyl-1,2,3,4-tetrahydronaphth-7-yl)-vinyl]-benzoesäure |
| 15 | $-CH_2-CH_2-$ | $CH_3$ | $CH_3$ | n-Oct | $CH_3$ | COOH | 130-131 | E | 20,83 | (E)-4-[2-Methyl-2-(1,1,4,4-tetramethyl-6-n-octyl-1,2,3,4-tetrahydronaphth-7-yl)-vinyl]-benzoesäure |
| 16 | $-CH_2-CH_2-$ | H | H | $CH_3$ | $CH_3$ | COOH | 233-234 | Z | | (Z)-4-[2-Methyl-2-(1,1-dimethyl-6-methyl-1,2,3,4-tetrahydronaphthyl-7-yl)-vinyl]-benzoesäure |

Beispiel 17

In eine Lösung von 4,0 g (E)-4-[2-Methyl-2-(1,1,4,4,6-pentamethyl-1,2,3,4-tetrahydronaphth-7-yl)-vinyl]-benzoesäure in 300 ml Ethanol wurde bei der Siedetemperatur des Reaktionsgemisches gasförmiger Chlorwasserstoff bis zur Sättigung eingeleitet. Anschließend wurde noch 4 h auf Siedetemperatur gehalten und nach dem Abkühlen mit Stickstoff gespült. Nach dem Eindampfen der Reaktionsmasse wurde mit 30 ml Methanol digeriert, abfiltriert und der Rückstand getrocknet. Man erhielt 3,6 g (E)-4-[2-Methyl-2-(1,1,4,4,6-pentamethyl-1,2,3,4-tetrahydronaphth-7-yl)-vinyl]-benzoesäureethylester, Schmp. 109 bis 110 °C, vgl. Beispiel 1.

Beispiel 18

Zu einer Suspension von 20,9 g (E)-4-[2-Methyl-2-(1,1,4,4-tetramethyl-1,2,3,4-tetrahydronaphth-7-yl)-vinyl]-benzoesäure (vgl. Beispiel 2) und 5,8 ml Pyridin in 200 ml wasserfreiem Tetrahydrofuran wurde rasch eine Lösung von 5,3 ml Thionylchlorid in 10 ml Tetrahydrofuran zugesetzt. Nach zweistündigem Rühren bei Raumtemperatur wurde der gebildete Niederschlag abfiltriert und das Filtrat mit Tetrahydrofuran auf ein Gesamtvolumen von 240 ml aufgefüllt.

100 ml dieser Lösung wurden innerhalb von 10 min nach und nach zu einer Suspension von 8,2 g p-Aminophenol in 100 ml Tetrahydrofuran zugesetzt. Anschließend wurde noch 20 h bei Raumtemperatur gerührt. Das Reaktiongemisch wurde in 1 l Wasser eingerührt, mit 2N Salzsäure angesäuert, der entstandene Niederschlag abfiltriert und aus Ethanol unter Zusatz von Wasser umkristallisiert.

Nach dem Abfiltrieren und Trocknen verblieben 11,1 g (E)-4-[2-Methyl-2-(1,1,4,4-tetramethyl-1,2,3,4-tetrahydronaphth-7-yl)vinyl]-benzoesäure-(4-hydroxyanilid), Schmp. 252 bis 253 °C.

$^{13}C$-NMR Spektrum ($d_6$-DMSO)

Das bei 17,30 ppm erscheinende Resonanzsignal für die Methylgruppe am Kohlenstoffatom 2 der olefinischen Ethylengruppe weist die erhaltene Verbindung als das E-(trans-)-Isomere aus.

Beispiele 19 bis 21

Die in der nachfolgenden Tabelle aufgeführten Verbindungen wurden analog Beispiel 18 erhalten :

(Siehe Tabelle Seite 13 f.)

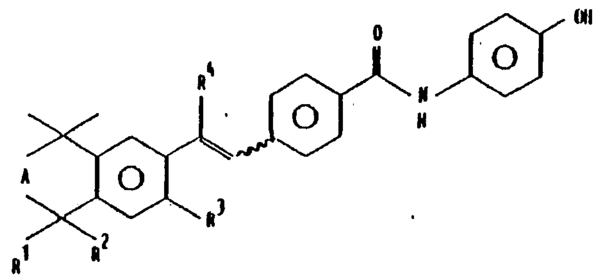

| Nr. | A | $R^1$ | $R^2$ | $R^3$ | $R^4$ | Fp [°C] | Stereochemie | $^{13}C$-Wert der Gruppe $R^4$ [ppm] | Name |
|---|---|---|---|---|---|---|---|---|---|
| 19 | $H_3C-CH$ | $-CH_3$ | $-CH_3$ | H | $-CH_3$ | 233-234 | E | 17,57 | (E)-4-[2-Methyl-2-(1,1,2,3,3-pentamethyl-indan-6-yl)-vinyl]-benzoesäure-(4-hydroxyanilid) |
| 20 | $-CH_2-CH_2-$ | $-CH_3$ | $-CH_3$ | $-CH_3$ | $-CH_3$ | 271-272 | E | 20,15 | (E)-4-[2-Methyl-2-(1,1,4,4,6-pentamethyl-1,2,3,4-tetrahydronaphth-7--yl)-vinyl]-benzoesäure-(4-hydroxyanilid) |
| 21 | $-CH_2-CH_2-$ | $-CH_3$ | $-CH_3$ | $-CH_2-CH_3$ | $-CH_3$ | 233-234 | E | 21,00 | (E)-4-[2-Methyl-(1,1,4,4-tetramethyl-6-ethyl-1,2,3,4-tetrahydronaphth-7-yl)-vinyl]-benzoesäure--(4-hydroxyanilid) |
| 22 | $-CH_2-CH_2-$ | $-CH_3$ | $-CH_3$ | $CH_3$<br>$-CH$<br>$CH_3$ | $-CH_3$ | 229-230 | E | 21,19 | (E)-4-[2-Methyl-2-(1,1,4,4-tetramethyl-6-isopropyl-1,2,3,4-tetrahydronaphth-7-yl)-vinyl]-abeenzoesäure-(4-hydroxyanilid) |
| 23 | $-CH_2-$ | $-CH_3$ | $-CH_3$ | H | $-CH_3$ | >300 | E | 17,56 | (E)-4-[2-Methyl-(1,1,3,3-tetramethyl-indan-6-yl)-vinyl]-benzoesäure-(4-hydroxyanilid) |

## Beispiel 24

Zu einer Suspension von 1,8 g (E)-4-[2-Methyl-2-(1,1,4,4-tetramethyl-1,2,3,4-tetrahydronaphth-7-yl)-vinyl]-benzoesäure (vgl. Beispiel 2) und 0,5 ml Pyridin in 15 ml Tetrahydrofuran wurde rasch eine Lösung von 0,5 ml Thioinyl-chlorid in 5 ml Tetrahydrofuran zugesetzt. Nach 2 h Rühren bei Raumtemperatur wurde der gebildete Niederschlag abfiltriert, das Filtrat zu einer Suspension aus 0,5 g wasserfreiem 5-Aminotetrazol und 0,5 g Pyridin in 20 ml Tetrahydrofuran zugefügt und anschließend noch 20 h bei Raumtemperatur gerührt. Die Reaktionsmasse wurde in 400 ml Wasser eingerührt, mit 2N Salzsäure angesäuert, der entstandene Niederschlag abfiltriert und mit Methanol und Petrolether gewaschen. Es verblieben 1,7 g (E)-4-[2-Methyl-2-(1,1,4,4-tetramethyl-1,2,3,4-tetrahydronaphth-7-yl)-vinyl]-N-(tetrazol-5-yl)-benzoesäureamid, Schmp. 289-290 °C.

C-NMR-Spektrum ($d_6$-DMSO)

Das bei 17,41 ppm erscheinende Resonanzsignal für die Methylgruppe am Kohlenstoffatom 2 der olefinischen Ethylengruppe weist die erhaltene Verbindung als das E-(trans-)-Isomere aus.

## Beispiele 25 bis 29

Die in der nachfolgenden Tabelle aufgeführten Verbindungen wurden analog Beispiel 1 erhalten :

(Siehe Tabelle Seite 15 f.)

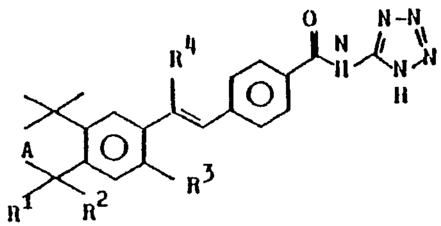

| Nr. | A | $R^1$ | $R^2$ | $R^3$ | $R^4$ | Fp [°C] | Stereo-chemie | $^{13}C$-Wert der Gruppe $R^4$ [ppm] | Name |
|---|---|---|---|---|---|---|---|---|---|
| 25 | $-CH_2-CH_2-$ | $-CH_3$ | $-CH_3$ | $-CH_3$ | $-CH_3$ | 287-288 | E | 20,17 | (E)-4-[2-Methyl-2-(1,1,4,6,-pentamethyl-1,2,3,4-tetrahydronaphth-7--yl)-vinyl]-N-(tetrazol-5-yl)-benzoesäureamid |
| 26 | $H_3C-CH<$ | $-CH_3$ | $-CH_3$ | H | $-CH_3$ | 285-286 | E | 17,76 | (E)-4-[2-Methyl-2-(1,1,2,3,3-pentamethyl-indan-6-yl)-vinyl]-N-(tetrazol-5-yl)-benzoesäureamid |
| 27 | $-CH_2-CH_2-$ | $-CH_3$ | $-CH_3$ | $-CH_2-CH_3$ | $-CH_3$ | 280-281 | E | 20,81 | (E)-4-[2-Methyl-(1,1,4,4-tetramethyl-6-ethyl-1,2,3,4-tetrahydronaphth-7-yl)-vinyl]-N-tetrazol--5-yl)-benzoesäureamid |
| 28 | $-CH_2-CH_2-$ | $-CH_3$ | $-CH_3$ | $-CH(CH_3)_2$ | $-CH_3$ | 296-297 | E | 21,26 | (E)-4-[2-Methyl-2-(1,1,4,4-tetramethyl-6-isopropyl-1,2,3,4-tetrahydronaphth-7-yl)-vinyl]-N-)tetrazol--5-yl)-benzoesäureamid |
| 29 | $-CH_2-$ | $-CH_3$ | $-CH_3$ | H | $-CH_3$ | 279-280 | E | 17,64 | (E)-4-[2-Methyl-(1,1,3,3-tetramethyl-indan-6-yl)-vinyl]-N-(tetrazol--5-yl)benzoesäureamid |

**Patentansprüche**

1. Phenylethylen-Derivate der Formel

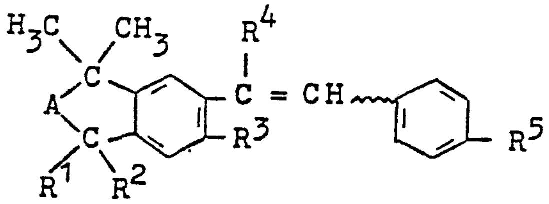

(I)

,

worin
A eine gegebenenfalls durch eine Methylgruppe substituierte Methylen- oder Ethylengruppe,
$R^1$ ein Wasserstoffatom oder eine Methylgruppe,
$R^2$ ein Wasserstoffatom oder eine Methylgruppe,
$R^3$ ein Wasserstoffatom oder eine $C_{1-8}$-Alkylgruppe,
$R^4$ eine $C_{1-4}$-Alkylgruppe und
$R^5$ eine p-Hydroxyphenylaminocarbonyl- oder Tetrazol-5-ylaminocarbonylgruppe oder-falls $R^3$ eine $C_{1-8}$-Alkylgruppe bedeutet — auch eine Carboxyl- oder $C_{2-4}$-Carbalkoxygruppe
darstellen, sowie gegebenenfalls deren Salze mit physiologisch verträglichen Basen.

2. Phenylethylen-Derivate der Formel I gemäß Anspruch 1, worin
A einen Ethylenrest
$R^1$ und $R^2$ Methylgruppen
$R^3$ eine $C_{1-8}$-Alkylgruppe
$R^4$ eine Methylgruppe und
$R^5$ eine Carboxyl- oder $C_{2-4}$-Carbalkoxygruppe
bedeuten, sowie deren Salze mit physiologisch verträglichen Säuren.

3. Phenylethylen-Derivate der Formel I gemäß Anspruch 1 oder 2, dadurch gekennzeichnet, daß die Phenylringe zueinander in trans-Stellung stehen.

4. (E)-4-[2-Methyl-2-(1,1,4,4-tetramethyl-6-(2-methylpropyl)-1,2,3,4-tetrahydronaphth-7-yl)-vinyl]-benzoesäure.

5. Verfahren zur Herstellung der Verbindungen der Formel I gemäß Anspruch 1, dadurch gekennzeichnet, daß man

a) eine Verbindung der Formel II

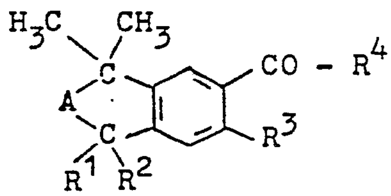

(II)

worin A und $R^1$-$R^4$ die in Anspruch 1 angegebene Bedeutung besitzen, mit einer Phosphorverbindung der Formel III

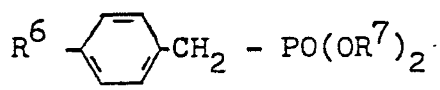

(III)

worin $R^6$ die für $R^5$ angegebene Bedeutung hat oder eine Cyanogruppe bedeutet und $R^7$ eine $C_{1-4}$-Alkylgruppe darstellt, nach Wittig-Horner umsetzt oder

b) eine Phosphoniumsalz der Formel IV

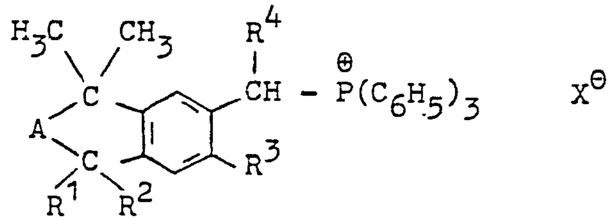

(IV)

worin A, $R^1$, $R^2$, $R^3$ und $R^4$ die in Anspruch 1 angegebene Bedeutung besitzen und X Chlor oder Brom bedeutet, mit einem Benzaldehyd-Derivat der Formel V

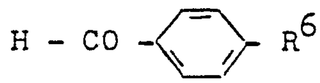 (V)

worin $R^6$ die oben angegebene Bedeutung besitzt, nach Wittig umsetzt sowie anschließend — falls $R^6$ keine Carboxylgruppe darstellt — die so erhaltene Verbindung gegebenenfalls verseift und die so oder direkt erhaltenen freien Säuren anschließend gewünschtenfalls mit einem $C_{1-3}$-Akohol, p-Hydroxyanilin oder 5-Aminotetrazol umsetzt und die so erhaltenen Verbindungen — falls gewünscht — in ihre Salze mit physiologisch verträglichen Basen überführt.

6. Arzneimittel, enthaltend eine Verbindung der Formel I gemäß Anspruch 1.

7. Verbindung der Formel I gemäß Anspruch 1 zur Verwendung bei der Bekämpfung von Krankheiten.

**Claims**

1. A phenylethylene derivative of the formula

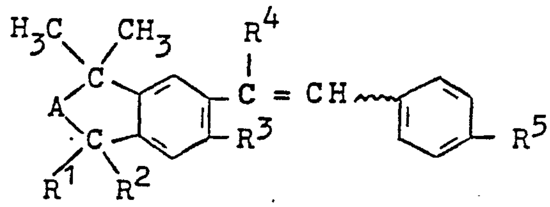 (I)

where A is methylene or ethylene which is unsubstituted or substituted by methyl, $R^1$ is hydrogen or methyl, $R^2$ is hydrogen or methyl, $R^3$ is hydrogen or $C_1$-$C_8$-alkyl, $R^4$ is $C_1$-$C_4$-alkyl, and $R^5$ is p-hydroxyphenylaminocarbonyl or tetrazol-5-ylaminocarbonyl and, if $R^3$ is $C_1$-$C_8$-alkyl, may furthermore be carboxyl or $C_2$-$C_4$-carbalkoxy, and, if desired, its salts with physiologically tolerated bases.

2. A phenylethylene derivative of the formula I as claimed in claim 1, where A is ethylen, $R^1$ and $R^2$ are each methyl, $R^3$ is $C_1$-$C_8$-alkyl, $R^4$ is methyl, and $R^5$ is carboxyl or $C_2$-$C_4$-carboalkoxy, and its salts with physiologically tolerated acids.

3. A phenylethylene derivative of the formula I as claimed in claim 1 or 2, wherein the phenyl rings are in the trans-position to one another.

4. E-4-[2-methyl-2-(1,1,4,4-tetramethyl-6-(2-methylpropyl)-1,2,3,4-tetrahydronaphth-7-yl)-vinyl]-benzoic acid.

5. A process for the preparation of a compound of the formula I as claimed in claim 1, wherein

a) a compound of the formula II

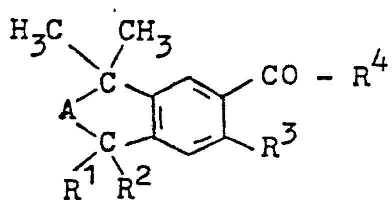 (II)

where A, $R^1$, $R^2$, $R^3$ and $R^4$ have the meanings given in claim 1, is subjected to a Wittig-Horner reaction with a phosphorus compound of the formula III

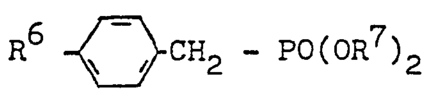 (III)

where $R^6$ has the meanings given for $R^5$ or is cyano, and $R^7$ is $C_1$-$C_4$-alkyl, or

b) a phosphonium salt of the formula IV

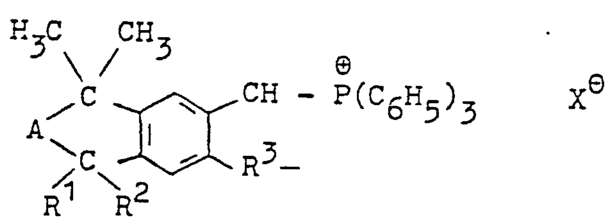 (IV)

where A, $R^1$, $R^2$, $R^3$, and $R^4$ have the meanings given in claim 1 and X is chlorine or bromine, is subjected to a Wittig reaction with a benzaldehyde derivative of the formula V

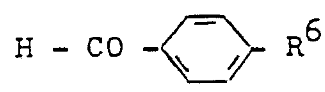

(V)

where $R^6$ has the above meanings,
and thereafter, if $R^6$ is not carboxyl, the resulting compound may, if desired, be hydrolyzed and the free acids, obtained either in this manner or directly, may, if desired, then be reacted with a $C_1$-$C_3$-alcohol, p-hydroxy-aniline or 5-aminotetrazole, and the compound thus obtained may, if desired, be converted into its salt with a physiologically tolerated base.

6. A pharmaceutical containing a compound of the formula I as claimed in claim 1.

7. A compound of the formula I as claimed in claim 1 for use in combating disorders.

**Revendications**

1. Dérivés de phényl-éthylène de la formule

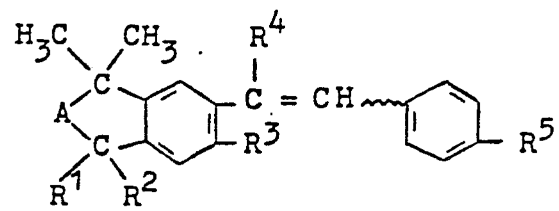

(I)

dans laquelle

A désigne un groupe méthylène ou éthylène éventuellement substitué par un radical méthyle,
$R^1$ désigne un atome d'hydrogène ou un radical méthyle,
$R^2$ désigne un atome d'hydrogène ou un radical méthyle,
$R^3$ désigne un atome d'hydrogène ou un radical alkyle en $C_1$ à $C_8$,
$R^4$ désigne un radical alkyle en $C_1$ à $C_4$,
$R^5$ désigne un groupe p-hydroxyphényl-aminocarbonyle ou tétrazol-5-yl-aminocarbonyle ou aussi, lorsque $R^3$ désigne un radical alkyle en $C_1$ à $C_8$, un groupe carboxyle ou carbalcoxy en $C_2$ à $C_4$,
ainsi qu'éventuellement les sels de bases physiologiquement acceptables de ces dérivés.

2. Dérivés de phényl-éthylène de la formule I selon la revendication 1, pour lesquels
A est un groupe éthylène,
$R^1$ et $R^2$ sont des radicaux méthyle,
$R^3$ est un radical alkyle en $C_1$ à $C_8$,
$R^4$ est un radical méthyle et
$R^5$ est un groupe carboxyle ou carbalcoxy en $C_2$ à $C_4$,
ainsi que les sels d'acides physiologiquement acceptables de ces dérivés.

3. Dérivés de phényl-éthylène de la formule I selon la revendication 1 ou 2, caractérisés en ce que les noyaux phényle se trouvent en position trans l'un par rapport à l'autre.

4. L'acide (E)-4-[2-méthyl-2-(1,1,4,4-tétraméthyl-6-(2-méthylpropyl)-1,2,3,4-tétrahydro-napht-7-yl)-vinyl]-benzoïque.

5. Procédé de préparation de composés de la formule I selon la revendication 1, caractérisé en ce que l'on fait réagir :

a) un composé de la formule II

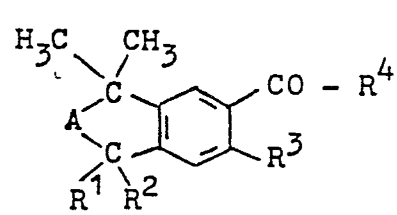

(II)

dans laquelle A et $R^1$ à $R^4$ possèdent la signification définie dans la revendication 1, et un dérivé du phosphore de la formule III

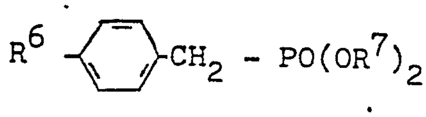

(III)

dans laquelle $R^6$ possède la signification définie pour $R^5$ ou désigne un groupe cyano et $R^7$ représente un radical alkyle en $C_1$ à $C_4$, par une réaction de Wittig-Horner,
ou

b) un sel de phosphonium de la formule IV

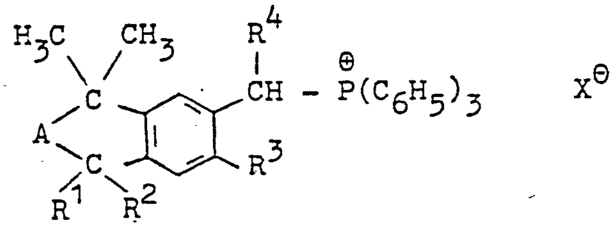

(IV)

dans laquelle A, $R^1$, $R^2$, $R^3$ et $R^4$ possèdent la signification définie dans la revendication 1 et X représente un atome de chlore ou de brome, et un dérivé du benzaldéhyde de la formule V

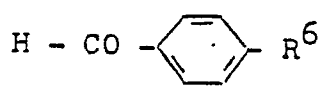

(V)

dans laquelle $R^6$ possède la signification définie ci-dessus, par une réaction de Wittig, le composé obtenu étant ensuite, si $R^6$ ne représente pas un groupe carboxyle, soumis éventuellement à une saponification, puis on fait éventuellement réagir l'acide libre obtenu de cette façon ou directement avec un alcool en $C_1$ à $C_3$, la para-hydroxy-aniline ou l'amino-5 tétrazole, les dérivés formés pouvant, si on le désire, être transformés en sels de bases physiologiquement acceptables.

6. Médicament, contenant un dérivé de la formule I selon la revendication 1.

7. Utilisation d'un composé de la formule I selon la revendication 1 pour le traitement de maladies.